# FASCICULE DE BREVET EUROPEEN

(11) **EP 3 615 499 B1**
(45) Date de publication et mention de la délivrance du brevet: **24.03.2021**
(21) Numéro de dépôt: 18725273.9
(22) Date de dépôt: 20.04.2018
(51) Int. Cl.: C07C 17/20, C07C 17/25, C07C 17/383, C07C 21/18, C01B 7/19

(54) **PROCÉDÉ DE PURIFICATION DU 1,1,1,2,2-PENTAFLUOROPROPANE**
VERFAHREN ZUR REINIGUNG VON 1,1,1,2,2-PENTAFLUORPROPEN
METHOD FOR PURIFYING 1,1,1,2,2-PENTAFLUOROPROPANE

(30) Priorité: 28.04.2017 FR 1753743
(43) Date de publication de la demande: 04.03.2020
(73) Titulaire: Arkema France, 92700 Colombes (FR)
(72) Inventeur: DEUR-BERT, Dominique, 69390 Charly (FR); GARRAIT, Dominique, 69390 Charly (FR); PIGAMO, Anne, 69340 Francheville (FR); WENDLINGER, Laurent, 69510 Soucieu en Jarrest (FR)
(74) Mandataire: Arkema Patent
(86) Numéro de dépôt international: PCT/FR2018/050994
(87) Numéro de publication internationale: WO 2018/197787

(56) Documents cités:
- WO-A1-2016/059322
- WO-A2-2012/067980
- WO-A2-2014/151448

## Description

### Domaine technique

La présente invention concerne un procédé de purification du 1,1,1,2,2-pentafluoropropane. En particulier, la présente invention concerne la purification du 1,1,1,2,2-pentafluoropropane dans le cadre d'un procédé de production du 2,3,3,3-tetrafluoropropène (HFO-1234yf).

### Arrière-plan technologique de l'invention

Dans un procédé de fluoration en phase gaz en présence d'acide fluorhydrique, les impuretés lourdes sont généralement contenues dans le flux d'acide fluorhydrique excédentaire qui est recyclé dans la boucle réactionnelle. Ce flux d'HF est obtenu par distillation, en pied de colonne de distillation, alors qu'en tête le composé fluoré désiré (HFO-1234yf) et l'HCl sont récupérés. De fort ratio molaire d'acide fluorhydrique étant mis en œuvre dans la boucle réactionnelle, ce flux d'acide fluorhydrique à recycler contient principalement de l'acide fluorhydrique.

Parmi les impuretés lourdes à éliminer, celles susceptibles de se déhydrochlorer ou de se déhydrofluorer pour donner naissance à des propynes halogénées sont particulièrement gênantes. Ces propynes sont réputées pour être extrêmement instables et par conséquence propices à la formation d'oligomères et de coke. Le recyclage et par conséquent l'accumulation de ces composés indésirables dans la boucle réactionnelle contribuent en partie à la détérioration de la stabilité (activité et sélectivité) du catalyseur. Ainsi, éviter leur accumulation dans la boucle réactionnelle permettrait donc de réduire la vitesse de cokage du catalyseur et ainsi d'améliorer la stabilité du catalyseur.

Une des possibilités pour éliminer ses impuretés est de procéder à une séparation de phase par décantation à froid (température < 20°C) sur tout ou partie de ce flux d'acide fluorhydrique recyclé pour obtenir une phase supérieure riche en HF et une phase riche en organique. La phase riche en organique (qui contient donc peu d'HF) peut alors être traitée par distillation pour récupérer les organiques d'intérêt (HCFO-1233xf par ex) et éliminer en pied des composés lourds. Ceci est notamment décrit dans les documents WO2013/130385 et WO2014/151448 qui décrivent une étape de décantation pour obtenir une phase riche en HF et une phase riche en organiques, dans le cadre de l'étape de fluoration en phase gaz du composé chloré en 2-chloro-3,3,3-trifluoropropène (HCFO-1233xf). Les documents WO 2016/059322 et WO 2012/067980 décrivent un procédé de production et/ou de purification du 2,3,3,3-tétrafluoropropène.

Cependant, lorsque le flux d'acide fluorhydrique excédentaire recyclé comprend trop d'acide fluorhydrique, la séparation des impuretés lourdes de l'acide fluorhydrique par décantation n'est pas efficace puisque le flux d'HF à recycler contient une concentration en HF supérieure ou voisine de celle pouvant être obtenue par décantation. La phase organique obtenue sera ainsi relativement faible par rapport à la phase riche en HF. En conséquence, la purge en organiques lourds indésirables peut être très peu performante voire inefficace en dépit des moyens mis en œuvre. Optionnellement, une purge directe à partir de ce flux d'HF excédentaire recyclé serait trop coûteuse car elle impliquerait de perdre beaucoup trop d'HF pour éliminer de faibles teneurs en organiques.

Il existe donc un besoin pour fournir un procédé de traitement des flux réactionnels générés dans la production du 2,3,3,3-tetrafluoropropène plus efficace et/ou moins couteux lors de sa mise en œuvre au niveau industriel.

### Résumé de l'invention

La présente invention permet de résoudre les problèmes décrits ci-dessus et observés dans l'art antérieur. La présente invention permet la mise en œuvre d'un procédé dans lequel une partie des composés lourds sont traités avec les impuretés légères plutôt qu'avec l'acide fluorhydrique excédentaire. Les composés sont ensuite séparés des impuretés plus légères. Ceci permet d'éliminer efficacement ces composés lourds, de contrôler leurs teneurs dans la boucle réactionnelle pour les maintenir à un faible niveau, et en conséquence d'améliorer la stabilité du catalyseur. Ce procédé est également plus économique et permet de minimiser les déchets. Le demandeur a notamment identifié trois impuretés lourdes, i.e. 2-chloro-1,1,1,3,3-pentafluoropropane (HCFC-235da), 1,2-dichloro-3,3,3-trifluoropropène (HCFO-1223xd), 2-chloro-1,3,3,3-tetrafluoropropène (HCFO-1224xe), lors de la mise en œuvre d'un procédé de production du 2,3,3,3-tetrafluoropropène. La nature de ces impuretés nécessite la mise en œuvre d'un procédé pour l'élimination de celles-ci tout en optimisant l'efficacité globale du procédé et son coût opérationnel.

Selon un premier aspect, l'invention fournit un procédé de purification d'un courant incluant 1,1,1,2,2-pentafluoropropane comprenant les étapes de :
i) fourniture d'un courant **A** comprenant 1,1,1,2,2-pentafluoropropane et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
ii) purification, de préférence par distillation, du courant **A** fourni en i) dans des conditions efficaces pour former un premier courant **A1** comprenant 1,1,1,2,2-pentafluoropropane, de préférence récupéré en tête de colonne de distillation, et un second courant **A2** comprenant ledit au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène, de préférence récupéré en pied de colonne de distillation.

Selon un mode de réalisation préféré, le courant **A** fourni à l'étape i) et ledit second courant **A2** de l'étape ii) comprennent au moins deux des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène, de préférence le courant **A** fourni à l'étape i) et ledit second courant **A2** de l'étape ii) comprennent 1,1,1,2,2-pentafluoropropane, 2-chloro-1,1,1,3,3-pentafluoropropane, 1,2-dichloro-3,3,3-trifluoropropène, 2-chloro-1,3,3,3-tetrafluoropropène.

Selon un mode de réalisation préféré, de l'acide fluorhydrique est également présent dans ledit courant **A** fourni à l'étape i).

Selon un mode de réalisation préféré, dans ledit premier courant **A1** obtenu à l'étape ii), le 1,1,1,2,2-pentafluoropropane est obtenu sous la forme d'un mélange azéotrope ou quasi-azéotrope, de préférence un mélange azéotrope ou quasi-azéotrope comprenant 1,1,1,2,2-pentafluoropropane et HF. En particulier, ledit mélange azéotrope ou quasi-azéotrope comprenant 1,1,1,2,2-pentafluoropropane et HF a une température d'ébullition comprise entre 0 et 60°C à une pression comprise entre 1 et 10 bara.

Selon un mode de réalisation préféré, le procédé comprend l'addition au courant **A** de l'étape i) d'un flux comprenant au moins 90% de 1,1,1,2,2-pentafluoropropane avant la mise en œuvre de l'étape ii).

Selon un mode de réalisation préféré, le courant **A** fourni à l'étape i) comprend également (E/Z)-1,3,3,3-tetrafluoropropène, 2-chloro-3,3,3-trifluoropropène, E-1-chloro-3,3,3-trifluoropropène, Z-1-chloro-3,3,3-trifluoropropène ou 1,1,1,3,3-pentafluoropropane.

Selon un mode de réalisation préféré, le (E/Z)-1,3,3,3-tetrafluoropropène, 2-chloro-3,3,3-trifluoropropène, E-1-chloro-3,3,3-trifluoropropène ou 1,1,1,3,3-pentafluoropropane est (sont) récupéré(s) dans ledit premier courant **A1** de l'étape (ii).

De préférence, le courant **A2** comprend Z-1-chloro-3,3,3-trifluoropropène.

Selon un autre aspect, l'invention fournit un procédé de production de 2,3,3,3-tetrafluoropropène comprenant les étapes :
a) fluoration en présence d'acide fluorhydrique d'un composé de formule (I) CH₍ₙ₊₂₎(X)ₘ-CHₚ(X)₍ₙ₊₁₎-CX₍₃₊ₚ₋ₘ₎ où X représente indépendamment F ou Cl ; n, m, p sont indépendamment les uns des autres 0 ou 1 avec (n+m) = 0 ou 1, (n+p) = 0 ou 1 et (m-p) = 0 ou 1, au moins un X étant Cl ; dans des conditions efficaces pour former un courant **C** comprenant 2,3,3,3-tetrafluoropropène, 1,1,1,2,2-pentafluoropropane, et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
b) purification, de préférence par distillation, du courant **C** obtenu à l'étape a) pour former un courant **C1** comprenant 2,3,3,3-tetrafluoropropène et un courant **C2** comprenant 1,1,1,2,2-pentafluoropropane, et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène,
c) mise en œuvre du procédé de purification du 1,1,1,2,2-pentafluoropropane selon la présente invention à partir du courant **C2** obtenu à l'étape b).

Selon un mode de réalisation préféré, le courant **C** obtenu à l'étape a) comprend également HF et ledit courant **C** obtenu à l'étape a) est purifié, de préférence distillé, préalablement à l'étape b) pour former un courant **C'** comprenant 2,3,3,3-tetrafluoropropène, 1,1,1,2,2-pentafluoropropane, et une partie desdits au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène et un courant **C"** comprenant HF et une partie desdits au moins un des composés sélectionnés parmi le groupe consistant en 2-chloro-1.1.1.3.3-pentafluoropropane, 1,2-dichloro-3,3,3-trifluoropropène, 2-chloro-1,3,3,3-tetrafluoropropène ; et l'étape b) est mise en œuvre à partir du courant **C'.**

Selon un mode de réalisation préféré, dans le courant **C',** la teneur totale en ladite partie desdits au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène est inférieure à 5% en poids sur base du poids total dudit courant **C'.**

Selon un autre aspect, l'invention fournit un procédé de traitement de l'acide fluorhydrique comprenant les étapes de
i') fourniture d'un mélange comprenant de l'acide fluorhydrique et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
ii') distillation du mélange fourni à l'étape i') dans des conditions efficaces pour former un courant **B1** comprenant ledit au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène et un courant **B2** comprenant de l'acide fluorhydrique.

### Brève description des figures

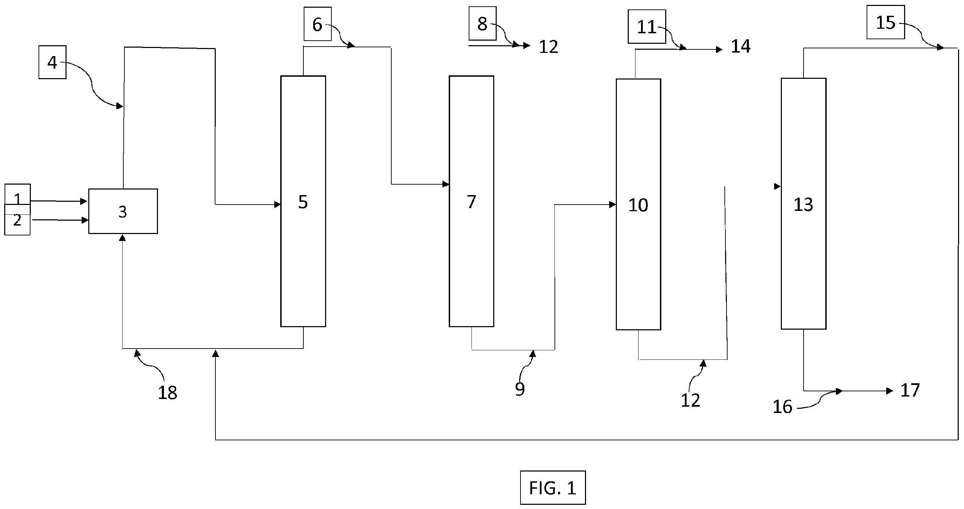

La figure 1 représente schématiquement un mode de réalisation particulier de la présente invention.

### Description détaillée de l'invention

Selon un premier aspect de la présente invention, un procédé de traitement de l'acide fluorhydrique est fourni. Ledit procédé comprend les étapes de :
i') fourniture d'un mélange comprenant de l'acide fluorhydrique et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
ii') purification, de préférence par distillation, du mélange fourni à l'étape i') dans des conditions efficaces pour former un courant **B1** comprenant ledit au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène et un courant **B2** comprenant de l'acide fluorhydrique.

De préférence, lorsque l'étape ii') est effectuée par distillation, la pression mise en œuvre lors de cette étape est de 2 à 8 bara, avantageusement de 3 à 6 bara, de préférence de 3,5 à 5,5 bara, en particulier l'étape ii') est mise en œuvre à une pression de 4 bara.

De préférence, lorsque l'étape ii') est effectuée par distillation, la température en tête de colonne de distillation est de -30°C à 20°C, avantageusement de -20°C à 10°C, de préférence de -15°C à 0°C.

De préférence, lorsque l'étape ii') est effectuée par distillation, la température en pied de colonne de distillation est de 10°C à 100°C, avantageusement de 20°C à 90°C, de préférence de 30°C à 80°C, en particulier de 40 à 70°C.

Selon un mode de réalisation préféré, ledit mélange de l'étape i') comprend également 2,3,3,3-tetrafluoropropène. De préférence, le 2,3,3,3-tetrafluoropropène est contenu dans le courant **B1** après la mise en œuvre de l'étape ii').

Selon un mode de réalisation préféré, ledit mélange de l'étape i') comprend également 1,1,1,2,2-pentafluoropropane. De préférence, le 1,1,1,2,2-pentafluoropropane est contenu dans le courant **B1** après la mise en œuvre de l'étape ii'). Lorsque le courant **B1** comprend 1,1,1,2,2-pentafluoropropane et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène, ledit courant **B1** correspond audit courant **A** selon la présente invention et, en particulier selon le présent procédé de purification de 1,1,1,2,2-pentafluoropropane selon la présente invention.

Selon un mode de réalisation préféré, ledit mélange de l'étape i') comprend également (E/Z)-1,3,3,3-tetrafluoropropène et HCl. De préférence, (E/Z)-1,3,3,3-tetrafluoropropène et HCl sont contenus dans le courant **B1** après la mise en œuvre de l'étape ii'). La teneur en HCl dans le courant **B1** peut être inférieure à 55% en poids, avantageusement inférieure à 50% en poids, de préférence inférieure à 45% en poids, plus préférentiellement inférieure à 40% en poids sur base du poids total dudit courant **B1.** La teneur en (E/Z)-1,3,3,3-tetrafluoropropène dans le courant **B1** peut être inférieure à 15% en poids, avantageusement inférieure à 10% en poids, de préférence inférieure à 5% en poids sur base du poids total dudit courant **B1.**

De préférence, ledit mélange de l'étape i') comprend également 2,3,3,3-tetrafluoropropène et celui est préférentiellement récupéré dans le courant **B1.** La teneur en 2,3,3,3-tetrafluoropropène dans le courant **B1** peut être inférieure à 50% en poids, avantageusement inférieure à 45% en poids, de préférence inférieure à 40% en poids, plus préférentiellement inférieure à 35% en poids, en particulier inférieure à 30% en poids, plus particulièrement inférieure à 25% en poids sur base du poids total dudit courant **B1.**

De préférence, ledit mélange de l'étape i') comprend également 1,1,1,2,2-pentafluoropropane et celui est préférentiellement récupéré dans le courant **B1.** La teneur en 1,1,1,2,2-pentafluoropropane dans le courant **B1** peut être inférieure à 50% en poids, avantageusement inférieure à 45% en poids, de préférence inférieure à 40% en poids, plus préférentiellement inférieure à 35% en poids, en particulier inférieure à 30% en poids sur base du poids total dudit courant **B1.**

De préférence, ledit mélange de l'étape i') comprend également 2-chloro-3,3,3-trifluoropropène. Dans ce cas, au moins une partie du 2-chloro-3,3,3-trifluoropropène est contenue dans le courant **B1** après la mise en œuvre de l'étape ii'). La teneur en 2-chloro-3,3,3-trifluoropropène dans ledit courant **B1** est inférieure à 20% en poids sur base du poids total dudit courant **B1**, avantageusement inférieure à 18% en poids, de préférence inférieure à 16% en poids, plus préférentiellement inférieure à 14% en poids, en particulier inférieure à 12% en poids, plus particulièrement inférieure à 10% en poids sur base du poids total dudit courant **B1.**

La teneur totale en ledit au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène dans le courant **B1** est inférieure à 5% en poids sur base du poids total du courant **B1**, avantageusement inférieure à 4% en poids, de préférence inférieure à 3% en poids, plus préférentiellement inférieure à 2% en poids, en particulier inférieure à 1% en poids, sur base du poids total du courant **B1.**

De préférence, la teneur en 2-chloro-1,1,1,3,3-pentafluoropropane dans ledit courant **B1** est inférieure à 4% en poids sur base du poids total dudit courant **B1**, avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **B1.**

De préférence, la teneur en 1,2-dichloro-3,3,3-trifluoropropène dans ledit courant **B1** est inférieure à 4% en poids sur base du poids total dudit courant **B1**, avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **B1.**

De préférence, la teneur en 2-chloro-1,3,3,3-tetrafluoropropène dans ledit courant **B1** est inférieure à 4% en poids sur base du poids total dudit courant **B1**, avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **B1.**

Une partie du 2-chloro-3,3,3-trifluoropropène peut également être contenue dans le courant **B2** après la mise en œuvre de l'étape ii').

Selon un mode de réalisation préféré, ledit mélange de l'étape i') comprend également (E/Z)-1-chloro-3,3,3-trifluoropropène et 1,1,1,3,3-pentafluoropropane. De préférence, le (E/Z)-1-chloro-3,3,3-trifluoropropène et 1,1,1,3,3-pentafluoropropane sont contenus dans le courant **B2** après la mise en œuvre de l'étape ii'). Néanmoins, une partie du (E/Z)-1-chloro-3,3,3-trifluoropropène peut être contenue dans le courant **B1** après la mise en œuvre de l'étape ii'). En particulier, une partie du E-1-chloro-3,3,3-trifluoro-1-propène (HCFO-1233zdE) peut être contenue dans ledit courant **B1.** La teneur en E-1-chloro-3,3,3-trifluoro-1-propène (HCFO-1233zdE) dans le courant **B1** peut être inférieure à 2% en poids sur base du poids total du courant **B1**, avantageusement inférieure à 1% en poids, de préférence inférieure à 0,5% en poids sur base du poids total du courant **B1.**

De préférence, la teneur en acide fluorhydrique est supérieure à 80% en mole dans ledit courant **B2** obtenu à l'étape ii'), avantageusement supérieure à 82% en mole, de préférence supérieure à 84% en mole, plus préférentiellement supérieure à 86% en mole, en particulier supérieure à 88% en mole, plus particulièrement supérieure à 90% en mole dans ledit courant **B2** obtenu à l'étape ii'). Cependant, une partie de l'acide fluorhydrique peut être présente dans le courant **B1.** La teneur en acide fluorhydrique dans le courant **B1** est inférieure à 15% en poids sur base du poids total du courant **B1**, avantageusement inférieure à 14% en poids, de préférence inférieure à 13% en poids, plus préférentiellement inférieure à 10% en poids sur base du poids total du courant **B1.**

Le courant **B1** obtenu à l'étape ii') peut être distillé pour éliminer l'acide chlorhydrique éventuellement présent. Cette distillation peut être effectuée dans des conditions telles que la température en tête de colonne de distillation peut être comprise entre -10°C et -70°C, plus préférentiellement entre -15°C et -65°C, en particulier entre -20°C et -60°C, plus particulièrement entre -25°C et -60°C, de manière privilégiée entre -30°C et -60°C ; et la pression en tête de colonne de distillation peut être comprise entre 2 et 20 bara, avantageusement entre 3 et 15 bara, de préférence entre 4 et 10 bara. Plus particulièrement, le courant **B1** peut être porté à une température inférieure à 100°C avant d'être distillé. Ainsi, le courant **B1** peut être à une température inférieure à 95°C, inférieure à 90°C, inférieure à 85°C, inférieure à 80°C, inférieure à 75°C, inférieure à 70°C, inférieure à 65°C, inférieure à 60°C, inférieure à 55°C, inférieure à 50°C, inférieure à 45°C, inférieure à 40°C, inférieure à 35°C, inférieure à 30°C, inférieure à 25°C, inférieure à 20°C, inférieure à 15°C, inférieure à 10°C, inférieure à 5°C ou inférieure à 0°C avant d'être distillé.

Selon un autre aspect, l'invention fournit un procédé de purification d'un courant comprenant du 1,1,1,2,2-pentafluoropropane. De préférence, le courant comprenant le 1,1,1,2,2-pentafluoropropane est un courant **A** comprenant 1,1,1,2,2-pentafluoropropane et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène. Ledit courant **A** peut correspondre au courant **B1** tel que décrit ci-dessus et obtenu par la mise en œuvre du procédé de traitement de l'acide fluorhydrique selon la présente invention. De préférence, ledit courant **A** peut correspondre au courant **B1** lorsque celui-ci ne contient pas d'acide chlorhydrique. Le courant **B1** peut être distillé pour éliminer l'acide chlorhydrique tel que décrit ci-dessus.

Ledit procédé de purification d'un courant comprenant du 1,1,1,2,2-pentafluoropropane comprend les étapes de :
i) fourniture d'un courant **A** comprenant 1,1,1,2,2-pentafluoropropane et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
ii) purification, de préférence par distillation, du courant **A** fourni en i) dans des conditions efficaces pour former un premier courant **A1** comprenant 1,1,1,2,2-pentafluoropropane, de préférence récupéré en tête de colonne de distillation, et un second courant **A2** comprenant ledit au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène, de préférence récupéré en pied de colonne de distillation.

Selon un mode de réalisation préféré, le courant **A** fourni à l'étape i) et ledit second courant **A2** de l'étape ii) comprennent au moins deux des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène, de préférence le courant **A** fourni à l'étape i) et ledit second courant **A2** de l'étape ii) comprennent 1,1,1,2,2-pentafluoropropane, 2-chloro-1,1,1,3,3-pentafluoropropane, 1,2-dichloro-3,3,3-trifluoropropène et 2-chloro-1,3,3,3-tetrafluoropropène.

De préférence, la teneur en 1,1,1,2,2-pentafluoropropane dans ledit courant **A** est inférieure à 75% en poids sur base du poids total dudit courant **A,** avantageusement inférieure à 74% en poids, de préférence inférieure à 73% en poids, plus préférentiellement inférieure à 72% en poids, en particulier inférieure à 71% en poids, plus particulièrement inférieure à 70% en poids sur base du poids total dudit courant **A.**

La teneur en ledit au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène dans le courant **A** est inférieure à 5% en poids sur base du poids total du courant **A,** avantageusement inférieure à 4% en poids, de préférence inférieure à 3% en poids, plus préférentiellement inférieure à 2% en poids, en particulier inférieure à 1% en poids sur base du poids total du courant **A.**

Selon un mode de réalisation préféré, de l'acide fluorhydrique est également présent dans ledit courant **A** fourni à l'étape i).

La teneur en acide fluorhydrique dans le courant **A** est inférieure à 15% en poids sur base du poids total du courant **A,** avantageusement inférieure à 14% en poids, de préférence inférieure à 13% en poids, plus préférentiellement inférieure à 12% en poids sur base du poids total du courant **A.**

Selon un mode de réalisation préféré, dans ledit premier courant **A1** obtenu à l'étape ii), le 1,1,1,2,2-pentafluoropropane est obtenu sous la forme d'un mélange azéotrope ou quasi-azéotrope, de préférence un mélange azéotrope ou quasi-azéotrope comprenant 1,1,1,2,2-pentafluoropropane et HF.

De préférence, la teneur en 1,1,1,2,2-pentafluoropropane dans le courant **A1** est supérieure à 50% en poids, avantageusement supérieure à 55% en poids, de préférence supérieure à 60% en poids sur base du poids total du courant **A1.** En particulier, la teneur en 1,1,1,2,2-pentafluoropropane dans le courant **A1** est 60 à 70% en poids de 1,1,1,2,2-pentafluoropropane.

Ledit premier courant **A1** peut éventuellement comprendre de faibles quantités en un des composés sélectionnés parmi le groupe consistant en 2-chloro-1,1,1,3,3-pentafluoropropane, 1,2-dichloro-3,3,3-trifluoropropène et 2-chloro-1,3,3,3-tetrafluoropropène. De préférence, la teneur totale en 2-chloro-1,1,1,3,3-pentafluoropropane, 1,2-dichloro-3,3,3-trifluoropropène, 2-chloro-1,3,3,3-tetrafluoropropène dans ledit premier courant **A1** obtenu à l'étape ii) est inférieure à 5% en poids sur base du poids total dudit premier courant **A1,** avantageusement inférieure à 4% en poids, de préférence inférieure à 3% en poids, plus préférentiellement inférieure à 2% en poids , en particulier inférieure à 1% en poids, plus particulièrement inférieure à 0,5% en poids sur base du poids total dudit premier courant **A1** obtenu à l'étape ii). Ainsi, même si le premier courant **A1** obtenu à l'étape ii) comprend des impuretés lourdes telles que 2-chloro-1,1,1,3,3-pentafluoropropane, 1,2-dichloro-3,3,3-trifluoropropène, 2-chloro-1,3,3,3-tetrafluoropropène, la teneur, exprimée en mole, de celles-ci dans ledit premier courant **A1** est inférieure à la teneur, exprimée en mole, de celles-ci dans ledit courant **A.**

De préférence, la teneur en 2-chloro-1,1,1,3,3-pentafluoropropane dans ledit courant **A1** est inférieure à 4% en poids sur base du poids total dudit courant **A1,** avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **A1.**

De préférence, la teneur en 1,2-dichloro-3,3,3-trifluoropropène dans ledit courant **A1** est inférieure à 4% en poids sur base du poids total dudit courant **A1,** avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **A1.**

De préférence, la teneur en 2-chloro-1,3,3,3-tetrafluoropropène dans ledit courant **A1** est inférieure à 4% en poids sur base du poids total dudit courant **A1,** avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **A1.**

De préférence, le procédé comprend l'addition au courant **A** de l'étape i) d'un flux comprenant au moins 70% de 1,1,1,2,2-pentafluoropropane avant la mise en œuvre de l'étape ii), avantageusement au moins 75% de 1,1,1,2,2-pentafluoropropane, de préférence au moins 80% de 1,1,1,2,2-pentafluoropropane, plus préférentiellement au moins 85% de 1,1,1,2,2-pentafluoropropane, en particulier au moins 90% de 1,1,1,2,2-pentafluoropropane, plus particulièrement au moins 95% de 1,1,1,2,2-pentafluoropropane en poids sur base du poids total dudit flux.

Selon un mode de réalisation préféré, le courant **A** fourni à l'étape i) comprend également (E/Z)-1,3,3,3-tetrafluoropropène, 2-chloro-3,3,3-trifluoropropène, E-1-chloro-3,3,3-trifluoropropène, Z-1-chloro-3,3,3-trifluoropropène ou 1,1,1,3,3-pentafluoropropane.

Selon un mode de réalisation préféré, le (E/Z)-1,3,3,3-tetrafluoropropène, 2-chloro-3,3,3-trifluoropropène, E-1-chloro-3,3,3-trifluoropropène ou 1,1,1,3,3-pentafluoropropane est (sont) récupéré(s) dans ledit premier courant **A1** de l'étape ii). De préférence, le Z-1-chloro-3,3,3-trifluoropropène est récupéré dans ledit courant **A2** de l'étape ii).

De préférence, la teneur en (E/Z)-1,3,3,3-tetrafluoropropène dans ledit premier courant **A1** de l'étape ii) est inférieure à 10% en poids sur base du poids total dudit premier courant **A1** de l'étape ii), avantageusement inférieure à 9% en poids, de préférence inférieure à 8% en poids, plus préférentiellement inférieure à 7% en poids, en particulier inférieure à 6% en poids, plus particulièrement inférieure à 5% en poids sur base du poids total dudit premier courant **A1** de l'étape ii).

De préférence, la teneur en 2-chloro-3,3,3-trifluoropropène dans ledit premier courant **A1** de l'étape ii) est inférieure à 20% en poids sur base du poids total dudit premier courant **A1** de l'étape ii), avantageusement inférieure à 19% en poids, de préférence inférieure à 18% en poids, plus préférentiellement inférieure à 17% en poids, en particulier inférieure à 16% en poids, plus particulièrement inférieure à 15% en poids sur base du poids total dudit premier courant **A1** de l'étape ii).

De préférence, la teneur en E-1-chloro-3,3,3-trifluoropropène dans ledit premier courant **A1** de l'étape ii) est inférieure à 5% en poids sur base du poids total dudit premier courant **A1** de l'étape ii), avantageusement inférieure à 4% en poids, de préférence inférieure à 3% en poids, plus préférentiellement inférieure à 2% en poids, en particulier inférieure à 1% en poids, plus particulièrement inférieure à 0.5% en poids sur base du poids total dudit premier courant **A1** de l'étape ii).

De préférence, la teneur en 1,1,1,3,3-pentafluoropropane dans ledit premier courant **A1** de l'étape ii) est inférieure à 5% en poids sur base du poids total dudit premier courant **A1** de l'étape ii), avantageusement inférieure à 4% en poids, de préférence inférieure à 3% en poids, plus préférentiellement inférieure à 2% en poids, en particulier inférieure à 1% en poids, plus particulièrement inférieure à 0.5% en poids sur base du poids total dudit premier courant **A1** de l'étape ii).

De préférence, la teneur en acide fluorhydrique dans ledit premier courant **A1** de l'étape ii) est inférieure à 15% en poids sur base du poids total dudit premier courant **A1** de l'étape ii), avantageusement inférieure à 14% en poids, de préférence inférieure à 13% en poids, plus préférentiellement inférieure à 12% en poids, en particulier inférieure à 11% en poids, plus particulièrement inférieure à 10% en poids sur base du poids total dudit premier courant **A1** de l'étape ii).

En particulier, ledit premier courant **A1** obtenu à l'étape ii) comprend un mélange azéotrope ou quasi-azéotrope comprenant HF, 1,1,1,2,2-pentafluoropropane et optionnellement (E/Z)-1,3,3,3-tetrafluoropropène, 2-chloro-3,3,3-trifluoropropène, E-1-chloro-3,3,3-trifluoropropène ou 1,1,1,3,3-pentafluoropropane, ledit mélange azéotrope ou quasi-azéotrope ayant une température d'ébullition comprise entre 0 et 60°C à une pression comprise entre 1 et 10 bara.

De préférence, l'étape ii) est mise œuvre par une distillation, la température en tête de colonne de distillation est comprise entre 0 et 60°C. En particulier, la pression est comprise entre 1 et 10 bara.

Ledit premier courant **A1** obtenu à l'étape ii) ainsi purifié peut être recyclé pour être utilisé dans un procédé de production du 2,3,3,3-tetrafluoropropène. En particulier, ledit premier courant **A1** obtenu à l'étape ii) peut alimenter un réacteur dans lequel une réaction de fluoration du 2-chloro-3,3,3-trifluoropropène est mise en œuvre. Plus particulièrement, ledit premier courant **A1** obtenu à l'étape ii) peut être mélangé avec un flux comprenant de l'acide fluorhydrique avant d'alimenter un réacteur dans lequel une réaction de fluoration du 2-chloro-3,3,3-trifluoropropène est mise en œuvre.

Selon un autre aspect de la présente invention, un procédé de production de 2,3,3,3-tetrafluoropropène est fourni. Ledit procédé comprend les étapes :
a) fluoration en présence d'acide fluorhydrique d'un composé de formule (I) CH₍ₙ₊₂₎(X)ₘ-CHₚ(X)₍ₙ₊₁₎-CX₍₃₊ₚ₋ₘ₎ où X représente indépendamment F ou Cl ; n, m, p sont indépendamment les uns des autres 0 ou 1 avec (n+m) = 0 ou 1, (n+p) = 0 ou 1 et (m-p) = 0 ou 1, au moins un X étant Cl dans des conditions efficaces pour former un courant **C** comprenant 2,3,3,3-tetrafluoropropène, 1,1,1,2,2-pentafluoropropane, et au moins un des composés sélectionné parmi le groupe consistant en 2-chloro-1,1,1,3,3-pentafluoropropane, 1,2-dichloro-3,3,3-trifluoropropène, 2-chloro-1,3,3,3-tetrafluoropropène ;
b) purification, de préférence par distillation, du courant **C** obtenu à l'étape a) pour former un courant **C1** comprenant 2,3,3,3-tetrafluoropropène et un courant **C2** comprenant 1,1,1,2,2-pentafluoropropane, et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
c) mise en œuvre du procédé de purification du 1,1,1,2,2-pentafluoropropane à partir du courant **C2** obtenu à l'étape b).

De préférence, le composé de formule (I) est sélectionné parmi le groupe consistant en 1,1,1,2,3-pentachloropropane, 1,1,2,3-tetrachloropropène, 2,3,3,3-tetrachloropropène, 2-chloro-3,3,3-trifluoropropène, 1,2-dichloro-3,3,3-trifluoropropane et 2-chloro-1,1,1,2-tetrafluoropropane, ou un mélange de ceux-ci. En particulier, le composé de formule (I) est 1,1,1,2,3-pentachloropropane, 2-chloro-3,3,3-trifluoropropène, 1,2-dichloro-3,3,3-trifluoropropane ou 1,1,2,3-tetrachloropropène.

De préférence, l'étape a) du présent procédé est effectuée en phase gazeuse. L'étape a) du présent procédé peut être mise en œuvre en présence ou non d'un catalyseur. Lorsqu'elle est réalisée en phase gazeuse et en présence de catalyseur, l'étape a) peut être mise en œuvre en présence d'un catalyseur à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine). On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb. On peut faire référence à cet égard au document WO 2007/079431 (en p.7, l.1-5 et 28-32), au document EP 939071 (paragraphe [0022]), au document WO 2008/054781 (en p.9 l.22-p.10 1.34), et au document WO 2008/040969 (revendication 1), auxquels il est fait expressément référence. Le catalyseur est de manière plus particulièrement préférée à base de chrome et il s'agit plus particulièrement d'un catalyseur mixte comprenant du chrome .Selon un mode de réalisation, on utilise pour l'une quelconque des étapes de réaction un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de nickel. Le métal peut être présent sous forme métallique ou sous forme de dérivé, par exemple un oxyde, halogénure ou oxyhalogénure. Ces dérivés sont de préférence obtenus par activation du métal catalytique. Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus. Le catalyseur peut comprendre du chrome et du nickel sous une forme activée ou non, sur un support qui a été soumis à une activation ou non. On peut se reporter au document WO 2009/118628 (notamment en p.4, l.30-p.7 l.16), auquel il est fait expressément référence ici. Un autre mode de réalisation préféré repose sur un catalyseur mixte contenant du chrome et au moins un co-catalyseur choisi parmi les sels de Co, Mn, Mg et Zn, de préférence Zn. Ledit co-catalyseur est de préférence présent dans une teneur de 1 à 10% en poids sur base du poids du catalyseur. Avant son utilisation, le catalyseur est de préférence soumis à une activation avec de l'air, de l'oxygène ou du chlore et/ou avec de l'HF. Par exemple, le catalyseur est de préférence soumis à une activation avec de l'air ou de l'oxygène et de l'HF à une température de 100 à 500°C, de préférence de 250 à 500°C et plus particulièrement de 300 à 400°C. La durée d'activation est de préférence de 1 à 200 h et plus particulièrement de 1 à 50 h. Cette activation peut être suivie d'une étape d'activation de fluoration finale en présence d'un agent d'oxydation, d'HF et de composés organiques. Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

En phase gazeuse, l'étape a) du présent procédé peut être mise en œuvre à une température de 200 à 450°C, avantageusement de 250 à 400°C, de préférence de 280 à 380°C. L'étape a) du présent procédé peut être mise en œuvre à un temps de contact de 3 à 100 s, avantageusement de 4 à 75 s, de préférence de 5 à 50 s. L'étape a) du présent procédé peut être mise en œuvre avec un rapport molaire HF/composé de formule (I) de 3:1 à 150 :1, avantageusement de 4 :1 à 125 :1, de préférence de 5 :1 à 100 :1. Le procédé, de préférence l'étape a), peut être mis en œuvre en présence d'un inhibiteur de polymérisation, de préférence sélectionné parmi le groupe consistant en p-methoxyphénol, t-amylphénol, limonène, d,1-limonene, quinones, hydroquinones, époxydes, amines et les mélanges de ceux-ci. L'étape a) du présent procédé peut être mise en œuvre en présence d'oxygène ou de chlore, avantageusement de 0.005 à 15 mole %, de préférence de 0.5 à 10 mole % d'oxygène ou de chlore par mole de composé de formule (I). L'étape a) est mise en œuvre à une pression 1 à 20 bara, de préférence de 2 à 15 bara, en particulier de 3 à 10 bara.

De préférence, l'étape a) du présent procédé est mise en œuvre en phase gazeuse en présence d'un catalyseur et d'un compose de formule (I) choisi parmi le groupe consistant en 1,1,1,2,3-pentachloropropane, 2-chloro-3,3,3-trifluoropropène, 1,2-dichloro-3,3,3-trifluoropropane ou 1,1,2,3-tetrachloropropène.

Alternativement, l'étape a) du présent procédé peut être mise en œuvre en phase liquide en présence d'un catalyseur. Le catalyseur peut être un acide de Lewis, un catalyseur contenant un halogénure d'un métal, en particulier un halogénure d'antimoine, étain, tantale, titane, d'un métal de transition tel que le molybdène, niobium, fer, césium, oxydes de métaux de transition, les halogénures de métaux du groupe IVb, les halogénures de métaux du groupe Vb, fluorure de chrome, oxydes de chrome fluorés ou un mélange de ceux-ci. Par exemple, le catalyseur peut être SbCl₅, SbCl₃, TiCl₄, SnCl₄, TaCl₅, NbCl₅, TiCl₄, FeCl₃, MoCl₆, CsCl, et les composés fluorés correspondants. Le catalyseur peut contenir un liquide ionique tel que décrit par exemple dans les demandes WO2008/149011 (en particulier de la page 4, ligne 1 à la page 6 ligne 15, inclus par référence) et WO01/81353, ainsi que la référence « liquid-phase HF Fluorination", Multiphase Homogeneous Catalysis, Ed. Wiley-VCH, (2002), 535. En phase liquide, l'étape a) peut être mise en œuvre à une température comprise entre 30 et 200°C, avantageusement entre 40°C et 170°C, de préférence entre 50 et 150°C. De préférence, le ratio molaire HF/composé de formule (I) peut être de 0,5 :1 à 50 :1, avantageusement de 3 :1 à 20 :1 et de préférence de 5 :1 à 15 :1.

De préférence, le courant **C** obtenu à l'étape a) comprend également HF et ledit courant **C** obtenu à l'étape a) est purifié, de préférence distillé, préalablement à l'étape b), étape notée a1). Le courant **C** peut correspondre au courant **B1** décrit ci-dessus lorsqu'il comprend 2,3,3,3-tetrafluoropropène, 1,1,1,2,2-pentafluoropropane, HF et au moins un des composés sélectionné parmi le groupe consistant en 2-chloro-1,1,1,3,3-pentafluoropropane, 1,2-dichloro-3,3,3-trifluoropropène, 2-chloro-1,3,3,3-tetrafluoropropène. Ainsi, la purification, de préférence la distillation, permet la formation d'un courant **C'** comprenant 2,3,3,3-tetrafluoropropène, 1,1,1,2,2-pentafluoropropane, et une partie desdits au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène et un courant **C"** comprenant HF et une partie desdits au moins un des composés sélectionné parmi le groupe consistant en 2-chloro-1,1,1,3,3-pentafluoropropane, 1,2-dichloro-3,3,3-trifluoropropène, 2-chloro-1,3,3,3-tetrafluoropropène.

De préférence, lorsque l'étape a1) est effectuée par distillation, la pression mise en œuvre lors de cette étape est de 2 à 8 bara, avantageusement de 3 à 6 bara, de préférence de 3,5 à 5,5 bara, en particulier l'étape a1) est mise en œuvre à une pression de 4 bara.

De préférence, lorsque l'étape a1) est effectuée par distillation, la température en tête de colonne de distillation est de -30°C à 20°C, avantageusement de -20°C à 10°C, de préférence de -15°C à 0°C.

De préférence, lorsque l'étape a1) est effectuée par distillation, la température en pied de colonne de distillation est de 10°C à 100°C, avantageusement de 20°C à 90°C, de préférence de 30°C à 80°C, en particulier de 40 à 70°C.

Ainsi, le courant **C'** peut être traité selon l'étape b) du présent procédé de production de 2,3,3,3-tetrafluoropropène.

De préférence, lorsque le courant **C** comprend HCl, ce dernier sera de préférence contenu dans le courant **C'.** En particulier, lorsque le courant **C'** comprend HCl, ledit courant **C'** est soumis à une étape de distillation a2) subséquente à l'étape a1) et préalable à l'étape b) pour former un courant **C''',** de préférence en tête de colonne distillation, comprenant HCl et un courant **C'''',** de préférence en pied de colonne de distillation, comprenant 2,3,3,3-tetrafluoropropène, 1,1,1,2,2-pentafluoropropane, et une partie desdits au moins un des composés sélectionné parmi le groupe consistant en 2-chloro-1,1,1,3,3-pentafluoropropane, 1,2-dichloro-3,3,3-trifluoropropène, 2-chloro-1,3,3,3-tetrafluoropropène. De préférence, la température en tête de colonne de distillation utilisée à l'étape a2) peut être compris entre - 10°C et -70°C, plus préférentiellement entre -15°C et -65°C, en particulier entre -20°C et -60°C, plus particulièrement entre -25°C et -60°C, de manière privilégiée entre -30°C et -60°C.

Selon un mode de réalisation préféré, la pression en tête de colonne de distillation utilisée à l'étape a2) peut être comprise entre 2 et 20 bara, avantageusement entre 3 et 15 bara, de préférence entre 4 et 10 bara. Plus particulièrement, le courant **C'** peut être porté à une température inférieure à 100°C avant d'être distillé. Ainsi, le courant **C'** peut être à une température inférieure à 95°C, inférieure à 90°C, inférieure à 85°C, inférieure à 80°C, inférieure à 75°C, inférieure à 70°C, inférieure à 65°C, inférieure à 60°C, inférieure à 55°C, inférieure à 50°C, inférieure à 45°C, inférieure à 40°C, inférieure à 35°C, inférieure à 30°C, inférieure à 25°C, inférieure à 20°C, inférieure à 15°C, inférieure à 10°C, inférieure à 5°C ou inférieure à 0°C avant d'être distillé.

En particulier, le courant **C''''** peut être traité selon l'étape b) du présent procédé de production de 2,3,3,3-tetrafluoropropène.

Ainsi, le présent procédé de production de 2,3,3,3-tetrafluoropropène comprend les étapes :
a) fluoration en présence d'acide fluorhydrique d'un composé de formule (I) CH₍ₙ₊₂₎(X)ₘ-CHₚ(X)₍ₙ₊₁₎-CX₍₃₊ₚ₋ₘ₎ où X représente indépendamment F ou Cl ; n, m, p sont indépendamment les uns des autres 0 ou 1 avec (n+m) = 0 ou 1, (n+p) = 0 ou 1 et (m-p) = 0 ou 1, au moins un X étant Cl dans des conditions efficaces pour former un courant **C** comprenant 2,3,3,3-tetrafluoropropène, 1,1,1,2,2-pentafluoropropane, et au moins un des composés sélectionné parmi le groupe consistant en 2-chloro-1,1,1,3,3-pentafluoropropane, 1,2-dichloro-3,3,3-trifluoropropène, 2-chloro-1,3,3,3-tetrafluoropropène et optionnellement HF et optionnellement HCl;
   a1) optionnellement purification, de préférence distillation, du courant **C** pour former un courant **C'** comprenant 2,3,3,3-tetrafluoropropène, 1,1,1,2,2-pentafluoropropane, et une partie desdits au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène et optionnellement HCl ; et un courant **C"** comprenant HF et une partie desdits au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
   a2) optionnellement purification, de préférence distillation, du courant **C'** obtenu à l'étape a1) pour former un courant **C''',** de préférence en tête de colonne distillation, comprenant HCl et un courant **C'''',** de préférence en pied de colonne de distillation, comprenant 2,3,3,3-tetrafluoropropène, 1,1,1,2,2-pentafluoropropane, et ladite une partie desdits au moins un des composés sélectionné parmi le groupe consistant en 2-chloro-1,1,1,3,3-pentafluoropropane, 1,2-dichloro-3,3,3-trifluoropropène, 2-chloro-1,3,3,3-tetrafluoropropène ;
b) purification, de préférence par distillation, du courant **C** obtenu à l'étape a) ou du courant **C'** obtenu à l'étape a1) ou du courant **C'''** obtenu à l'étape a2) pour former un courant **C1** comprenant 2,3,3,3-tetrafluoropropène et un courant **C2** comprenant 1,1,1,2,2-pentafluoropropane, et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
c) mise en œuvre du procédé de purification du 1,1,1,2,2-pentafluoropropane selon la présente invention à partir du courant **C2** obtenu à l'étape b) et purification, de préférence par distillation, du courant **C2** pour former un premier courant **C3** comprenant 1,1,1,2,2-pentafluoropropane, de préférence récupéré en tête de colonne de distillation, et un second courant **C4** comprenant ledit au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène, de préférence récupéré en pied de colonne de distillation. De préférence, lesdits courants **C3** et **C4** correspondent respectivement aux courants **A1** et **A2** décrits en relation avec le procédé de purification du 1,1,1,2,2-pentafluoropropane selon la présente invention.

Selon un mode de réalisation préféré, la température en tête de colonne de distillation à l'étape b) est comprise entre 0°C et 50°C. De préférence, la pression en tête de colonne de distillation à l'étape b) est comprise entre 3 et 12 bara.

De préférence, dans le courant **C'** et/ou le courant **C'''',** la teneur en ladite partie desdits au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène est inférieure à 10% en poids sur base du poids total dudit courant **C'** ou **C'''',** avantageusement inférieure à 9% en poids sur base du poids total dudit courant **C'** ou **C'''',** de préférence inférieure à 8% en poids sur base du poids total dudit courant **C'** ou **C'''',** plus préférentiellement inférieure à 7% en poids sur base du poids total dudit courant **C'** ou **C'''',** en particulier inférieure à 6% en poids sur base du poids total dudit courant **C'** ou **C'''',** plus particulièrement inférieure à 5% en poids sur base du poids total dudit courant **C'** ou **C'''',** de manière privilégiée inférieure à 4% en poids sur base du poids total dudit courant **C'** ou **C'''',** de manière plus privilégiée inférieure à 3% en poids sur base du poids total dudit courant **C'** ou **C''''.**

Le courant **C1** peut comprendre au moins 40% en poids de 2,3,3,3-tetrafluoropropène, avantageusement au moins 45% en poids, de préférence au moins 50% en poids, plus préférentiellement au moins 55% en poids, en particulier au moins 60% en poids, plus particulièrement au moins 65% en poids, de manière privilégiée au moins 70% en poids sur base du poids total du courant **C1.**

Le courant **C1** peut éventuellement comprendre de faibles quantités de 1,1,1,2,2-pentafluoropropane. De préférence, la teneur en 1,1,1,2,2-pentafluoropropane dans ledit courant **C1** est inférieure à 40% en poids sur base du poids total dudit courant **C1,** avantageusement inférieure à 35% en poids, de préférence inférieure à 30% en poids, plus préférentiellement inférieure à 25% en poids, en particulier inférieure à 22% en poids, plus particulièrement inférieure à 20% en poids sur base du poids total dudit courant **C1.**

Le courant **C1** peut éventuellement comprendre de faibles quantités d'acide fluorhydrique. De préférence, la teneur en acide fluorhydrique dans ledit courant **C1** est inférieure à 20% en poids sur base du poids total dudit courant **C1,** avantageusement inférieure à 18% en poids, de préférence inférieure à 16% en poids, plus préférentiellement inférieure à 14% en poids, en particulier inférieure à 12% en poids, plus particulièrement inférieure à 10% en poids sur base du poids total dudit courant **C1.**

Comme mentionné ci-dessus, le courant **C2** comprend 1,1,1,2,2-pentafluoropropane, et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène.

De préférence, la teneur en 1,1,1,2,2-pentafluoropropane dans le courant **C2** est inférieure à 75% en poids sur base du poids total dudit courant **C2,** avantageusement inférieure à 74% en poids, de préférence inférieure à 73% en poids, plus préférentiellement inférieure à 72% en poids, en particulier inférieure à 71% en poids, plus particulièrement inférieure à 70% en poids sur base du poids total dudit courant **C2.**

De préférence, la teneur totale en 2-chloro-1,1,1,3,3-pentafluoropropane, 1,2-dichloro-3,3,3-trifluoropropène, 2-chloro-1,3,3,3-tetrafluoropropène dans ledit premier courant **C2** est inférieure à 5% en poids sur base du poids total dudit premier courant **C2,** avantageusement inférieure à 4% en poids, de préférence inférieure à 3% en poids, plus préférentiellement inférieure à 2% en poids, en particulier inférieure à 1% en poids, plus particulièrement inférieure à 0,5% en poids sur base du poids total dudit premier courant **C2.**

De préférence, la teneur en 2-chloro-1,1,1,3,3-pentafluoropropane dans ledit courant **C2** est inférieure à 4% en poids sur base du poids total dudit courant **C2,** avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **C2.**

De préférence, la teneur en 1,2-dichloro-3,3,3-trifluoropropène dans ledit courant **C2** est inférieure à 4% en poids sur base du poids total dudit courant **C2,** avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **C2.**

De préférence, la teneur en 2-chloro-1,3,3,3-tetrafluoropropène dans ledit courant **C2** est inférieure à 4% en poids sur base du poids total dudit courant **C2,** avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **C2.**

Comme décrit ci-dessus en relation avec le procédé de purification du 1,1,1,2,2-pentafluoropropane, de l'acide fluorhydrique peut être présent dans le courant **C, C', C'''', C2, C3** et **C4.** De préférence, la teneur en acide fluorhydrique dans le courant **C2** est inférieure à 15% en poids sur base du poids total du courant **C2,** avantageusement inférieure à 14% en poids, de préférence inférieure à 13% en poids, plus préférentiellement inférieure à 12% en poids sur base du poids total du courant **C2.** Selon un mode de réalisation préféré, ledit premier courant **C3** comprend un mélange azéotrope ou quasi-azéotrope, de préférence un mélange azéotrope ou quasi-azéotrope comprenant 1,1,1,2,2-pentafluoropropane et HF.

De préférence, la teneur en 1,1,1,2,2-pentafluoropropane dans le courant **C3** est supérieure à 50% en poids, avantageusement supérieure à 55% en poids, de préférence supérieure à 60% en poids sur base du poids total du courant **C3.** En particulier, la teneur en 1,1,1,2,2-pentafluoropropane dans le courant **C3** est 60 à 70% en poids de 1,1,1,2,2-pentafluoropropane.

Ledit courant **C3** peut éventuellement comprendre de faibles quantités en un des composés sélectionnés parmi le groupe consistant en 2-chloro-1,1,1,3,3-pentafluoropropane, 1,2-dichloro-3,3,3-trifluoropropène et 2-chloro-1,3,3,3-tetrafluoropropène. De préférence, la teneur totale en 2-chloro-1,1,1,3,3-pentafluoropropane, 1,2-dichloro-3,3,3-trifluoropropène, 2-chloro-1,3,3,3-tetrafluoropropène dans ledit premier courant **C3** est inférieure à 5% en poids sur base du poids total dudit courant **C3,** avantageusement inférieure à 4% en poids, de préférence inférieure à 3% en poids, plus préférentiellement inférieure à 2% en poids , en particulier inférieure à 1% en poids, plus particulièrement inférieure à 0,5% en poids sur base du poids total dudit courant **C3.**

De préférence, la teneur en 2-chloro-1,1,1,3,3-pentafluoropropane dans ledit courant **C3** est inférieure à 4% en poids sur base du poids total dudit courant **C3,** avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **C3.**

De préférence, la teneur en 1,2-dichloro-3,3,3-trifluoropropène dans ledit courant **C3** est inférieure à 4% en poids sur base du poids total dudit courant **C3,** avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **C3.**

De préférence, la teneur en 2-chloro-1,3,3,3-tetrafluoropropène dans ledit courant **C3** est inférieure à 4% en poids sur base du poids total dudit courant **C3,** avantageusement inférieure à 3% en poids, de préférence inférieure à 2% en poids, plus préférentiellement inférieure à 1% en poids, en particulier inférieure à 0,8% en poids, plus particulièrement inférieure à 0,5% en poids sur base du poids total dudit courant **C3.**

De préférence, le procédé comprend l'addition au courant **C2** de l'étape i) d'un flux comprenant au moins 70% de 1,1,1,2,2-pentafluoropropane avant la mise en œuvre de l'étape ii), avantageusement au moins 75% de 1,1,1,2,2-pentafluoropropane, de préférence au moins 80% de 1,1,1,2,2-pentafluoropropane, plus préférentiellement au moins 85% de 1,1,1,2,2-pentafluoropropane, en particulier au moins 90% de 1,1,1,2,2-pentafluoropropane, plus particulièrement au moins 95% de 1,1,1,2,2-pentafluoropropane en poids sur base du poids total dudit flux.

Selon un mode de réalisation préféré, le courant **C, C', C'''', C2** et **C3** comprennent également (E/Z)-1,3,3,3-tetrafluoropropène, 2-chloro-3,3,3-trifluoropropène, E-1-chloro-3,3,3-trifluoropropène ou 1,1,1,3,3-pentafluoropropane.

Selon un mode de réalisation préféré, le (E/Z)-1,3,3,3-tetrafluoropropène, 2-chloro-3,3,3-trifluoropropène, E-1-chloro-3,3,3-trifluoropropène ou 1,1,1,3,3-pentafluoropropane est (sont) récupéré(s) dans ledit courant **C3.**

De préférence, la teneur en (E/Z)-1,3,3,3-tetrafluoropropène dans ledit courant **C3** est inférieure à 10% en poids sur base du poids total dudit courant **C3,** avantageusement inférieure à 9% en poids, de préférence inférieure à 8% en poids, plus préférentiellement inférieure à 7% en poids, en particulier inférieure à 6% en poids, plus particulièrement inférieure à 5% en poids sur base du poids total dudit courant **C3.**

De préférence, la teneur en 2-chloro-3,3,3-trifluoropropène dans ledit courant **C3** est inférieure à 20% en poids sur base du poids total dudit courant **C3,** avantageusement inférieure à 19% en poids, de préférence inférieure à 18% en poids, plus préférentiellement inférieure à 17% en poids, en particulier inférieure à 16% en poids, plus particulièrement inférieure à 15% en poids sur base du poids total dudit courant **C3.**

De préférence, la teneur en E-1-chloro-3,3,3-trifluoropropène dans ledit courant **C3** est inférieure à 5% en poids sur base du poids total dudit courant **C3,** avantageusement inférieure à 4% en poids, de préférence inférieure à 3% en poids, plus préférentiellement inférieure à 2% en poids, en particulier inférieure à 1% en poids, plus particulièrement inférieure à 0.5% en poids sur base du poids total dudit courant **C3.**

De préférence, la teneur en 1,1,1,3,3-pentafluoropropane dans ledit courant **C3** est inférieure à 5% en poids sur base du poids total dudit courant **C3,** avantageusement inférieure à 4% en poids, de préférence inférieure à 3% en poids, plus préférentiellement inférieure à 2% en poids, en particulier inférieure à 1% en poids, plus particulièrement inférieure à 0.5% en poids sur base du poids total dudit courant **C3.**

De préférence, la teneur en acide fluorhydrique dans ledit courant **C3** est inférieure à 15% en poids sur base du poids total dudit courant **C3,** avantageusement inférieure à 14% en poids, de préférence inférieure à 13% en poids, plus préférentiellement inférieure à 12% en poids, en particulier inférieure à 11% en poids, plus particulièrement inférieure à 10% en poids sur base du poids total dudit courant **C3.**

En particulier, ledit courant **C3** comprend un mélange azéotrope ou quasi-azéotrope comprenant HF, 1,1,1,2,2-pentafluoropropane et optionnellement (E/Z)-1,3,3,3-tetrafluoropropène, 2-chloro-3,3,3-trifluoropropène, E-1-chloro-3,3,3-trifluoropropène ou 1,1,1,3,3-pentafluoropropane, ledit mélange ayant une température d'ébullition comprise entre 0 et 60°C à une pression comprise entre 1 et 10 bara. De préférence, l'étape c) est mise œuvre par une distillation, la température en tête de colonne de distillation est comprise entre 0 et 60°C. En particulier, la pression est comprise entre 1 et 10 bara.

Ledit courant **C3** ainsi purifié peut être recyclé à l'étape a) du présent procédé de production du 2,3,3,3-tetrafluoropropène.

Selon un mode de réalisation préféré, le courant **C, C', C'''', C2** et **C4** comprennent Z-1-chloro-3,3,3-trifluoropropène.

De préférence, au moins 95% du cis-1-chloro-3,3,3-trifluoro-1-propène (HCFO-1233zdZ) contenu dans le courant **C2** est récupéré dans le courant **C4,** avantageusement au moins 96%, de préférence au moins 97%, plus préférentiellement au moins 98%, en particulier au moins 99%, plus particulièrement au moins 99,5% du cis-1-chloro-3,3,3-trifluoro-1-propène (HCFO-1233zdZ) contenu dans le courant **C2** est récupéré dans le courant **C4.**

De préférence, au moins 80% du 2-chloro-1,1,1,3,3-pentafluoropropane (HCFC-235da) contenu dans le courant **C2** est récupéré dans le courant **C4,** avantageusement au moins 85%, de préférence au moins 90%, plus préférentiellement au moins 93%, en particulier au moins 94%, plus particulièrement au moins 95% du 2-chloro-1,1,1,3,3-pentafluoropropane (HCFC-235da) contenu dans le courant **C2** est récupéré dans le courant **C4.** En particulier, de 90% à 98% du 2-chloro-1,1,1,3,3-pentafluoropropane (HCFC-235da) contenu dans le courant **C2** est récupéré dans le courant **C4.**

De préférence, au moins 80% du 1,2-dichloro-3,3,3-trifluoropropène (HCFO-1223xd) contenu dans le courant **C2** est récupéré dans le courant **C4** avantageusement au moins 85%, de préférence au moins 90%, plus préférentiellement au moins 93%, en particulier au moins 94%, plus particulièrement au moins 95% du 1,2-dichloro-3,3,3-trifluoropropène (HCFO-1223xd) contenu dans le courant **C2** est récupéré dans le courant **C4.** En particulier, de 90% à 98% du 1,2-dichloro-3,3,3-trifluoropropène (HCFO-1223xd) contenu dans le courant **C2** est récupéré dans le courant **C4.**

De préférence, au moins 80% du 2-chloro-1,3,3,3-tetrafluoropropène (HCFO-1224xe) contenu dans le courant **C2** est récupéré dans le courant **C4,** avantageusement au moins 85%, de préférence au moins 90%, plus préférentiellement au moins 93%, en particulier au moins 94%, plus particulièrement au moins 95% du 2-chloro-1,3,3,3-tetrafluoropropène (HCFO-1224xe) contenu dans le courant **C2** est récupéré dans le courant **C4.** En particulier, de 90% à 98% du 2-chloro-1,3,3,3-tetrafluoropropène (HCFO-1224xe) contenu dans le courant **C2** est récupéré dans le courant **C4.**

De préférence, ledit courant **C4** comprend de 1 à 25% en poids de 2-chloro-1,3,3,3-tetrafluoropropène (HCFO-1224xe), avantageusement de 5 à 20% en poids, de préférence de 7 à 18% en poids, en particulier de 10 à 15% en poids de 2-chloro-1,3,3,3-tetrafluoropropène (HCFO-1224xe) sur base du poids total dudit courant **C4.**

De préférence, ledit courant **C4** comprend de 40 à 85% en poids de 1,2-dichloro-3,3,3-trifluoropropène (HCFO-1223xd), avantageusement de 45 à 80% en poids, de préférence de 50 à 75% en poids, en particulier de 55 à 70% en poids de 1,2-dichloro-3,3,3-trifluoropropène (HCFO-1223xd) sur base du poids total dudit courant **C4.**

De préférence, ledit courant **C4** comprend de 1 à 25% en poids de 2-chloro-1,1,1,3,3-pentafluoropropane (HCFC-235da), avantageusement de 5 à 20% en poids, de préférence de 7 à 18% en poids, en particulier de 10 à 15% en poids de 2-chloro-1,1,1,3,3-pentafluoropropane (HCFC-235da) sur base du poids total dudit courant **C4.**

De préférence, ledit courant **C4** comprend de 0,05 à 10% en poids de cis-1-chloro-3,3,3-trifluoro-1-propène (HCFO-1233zdZ), avantageusement de 0,1 à 8% en poids, de préférence de 0,5 à 7% en poids, en particulier de 1 à 5% en poids de cis-1-chloro-3,3,3-trifluoro-1-propène (HCFO-1233zdZ) sur base du poids total dudit courant **C4.**

Ainsi, ledit courant **C4** peut comprendre de 1 à 5% en poids de cis-1-chloro-3,3,3-trifluoro-1-propène (HCFO-1233zdZ), de 10% à 15% en poids de 2-chloro-1,1,1,3,3-pentafluoropropane (HCFC-235da), de 55 à 70% en poids de 1,2-dichloro-3,3,3-trifluoropropène (HCFO-1223xd), de 10 à 15% en poids de 2-chloro-1,3,3,3-tetrafluoropropène (HCFO-1224xe).

De préférence, le courant **C4** comprend moins de 2% en poids d'acide fluorhydrique sur base du poids total du courant **C4,** avantageusement moins de 1% en poids, de préférence moins de 0,5% en poids, en particulier moins de 0,1% en poids d'acide fluorhydrique sur base du poids total du courant **C4.**

La figure 1 représente schématiquement un procédé de production du 2,3,3,3-tetrafluoropropène incluant le procédé de purification du 1,1,1,2,2-pentafluoropropane et le procédé de traitement de l'acide fluorhydrique selon la présente invention. L'acide fluorhydrique 1 est mis en contact avec du 1,1,1,2,3-pentachloropropane (HCC-240db) 2 dans un ou plusieurs réacteurs 3. La mise en contact est effectuée dans des conditions efficaces pour former un mélange **C** comprenant 2,3,3,3-tetrafluoropropène, 1,1,1,2,2-pentafluoropropane, HCl, HF, 2-chloro-1,1,1,3,3-pentafluoropropane, 1,2-dichloro-3,3,3-trifluoropropène et 2-chloro-1,3,3,3-tetrafluoropropène. Ledit mélange est récupéré en sortie de réacteur 3 et acheminé vers une colonne de distillation 5 par le conduit 4. Le mélange **C** est distillé de sorte à former un courant **C'** comprenant 2,3,3,3-tetrafluoropropène, 1,1,1,2,2-pentafluoropropane, HCl, 2-chloro-1,1,1,3,3-pentafluoropropane, 1,2-dichloro-3,3,3-trifluoropropène et 2-chloro-1,3,3,3-tetrafluoropropène et un courant **C"** comprenant HF, 2-chloro-1,1,1,3,3-pentafluoropropane, 1,2-dichloro-3,3,3-trifluoropropène et 2-chloro-1,3,3,3-tetrafluoropropène. Le courant **C',** récupéré en tête de colonne de distillation 5, est acheminé par la conduite 6 vers la colonne de distillation 7. Le courant **C",** récupéré en pied de colonne de distillation 5, est acheminé par la conduite 18 vers le réacteur 3 pour être recyclé dans la réaction de fluoration du 1,1,1,2,3-pentachloropropane après une éventuelle purification. Le courant **C'** est distillé en 7 pour former en tête de colonne de distillation un courant **C'''** comprenant HCl qui peut être éventuellement acheminé par la conduite 8 vers un dispositif de purification 12. Un courant **C''''** est récupéré en pied de colonne de distillation et est acheminé par la conduite 9 vers la colonne de distillation 10. Le courant **C''''** comprend 2,3,3,3-tetrafluoro-1-propène, 1,1,1,2,2-pentafluoropropane, 2-chloro-1,1,1,3,3-pentafluoropropane, 1,2-dichloro-3,3,3-trifluoropropène et 2-chloro-1,3,3,3-tetrafluoropropène. La colonne de distillation 10 permet la séparation entre d'un côté le 2,3,3,3-tetrafluoropropène (courant **C1**) et d'un autre côté le 1,1,1,2,2-pentafluoropropane, 2-chloro-1,1,1,3,3-pentafluoropropane, 1,2-dichloro-3,3,3-trifluoropropène et 2-chloro-1,3,3,3-tetrafluoropropène (courant **C2**). Le courant **C1** récupéré en tête de colonne de distillation est acheminé vers le dispositif de purification 14 par la conduite 11. Le courant **C2** est acheminé vers la colonne de distillation 13 par la conduite 12. La colonne de distillation 13 permet la séparation entre d'un côté le 1,1,1,2,2-pentafluoropropane (courant **C3**) et d'un autre côté le 2-chloro-1,1,1,3,3-pentafluoropropane, 1,2-dichloro-3,3,3-trifluoropropène et 2-chloro-1,3,3,3-tetrafluoropropène (courant **C4**). Le courant **C3** est recyclé vers le réacteur 3 par les conduites 15 et 18. Le courant **C4** est récupéré en pied de colonne de distillation et par exemple acheminé vers un incinérateur 17 par la conduite 16. Le courant **C3** comprend de faibles quantités de 2-chloro-1,1,1,3,3-pentafluoropropane, 1,2-dichloro-3,3,3-trifluoropropène et 2-chloro-1,3,3,3-tetrafluoropropène comme expliqué dans la présente demande. La présente invention permet de purifier le flux de 1,1,1,2,2-pentafluoropropane de composés lourds et ainsi améliorer le procédé global de production du 2,3,3,3-tetrafluoropropène en évitant l'accumulation des composés tels que 2-chloro-1,1,1,3,3-pentafluoropropane, 1,2-dichloro-3,3,3-trifluoropropène et 2-chloro-1,3,3,3-tetrafluoropropène dans la boucle réactionnelle. Ceci est en outre réalisé en optimisant la récupération d'acide fluorhydrique n'ayant pas réagi.

### Exemple

### Exemple 1

Un courant comprenant 87% 1,1,1,2,2-pentafluoropropane, 10% d'HF, 1% de 2-chloro-1,1,1,3,3-pentafluoropropane, 0,8 % de 1,2-dichloro-3,3,3-trifluoropropène et 0,5% de 2-chloro-1,3,3,3-tetrafluoropropène en poids est distillé dans des conditions telles que la température en tête de colonne de distillation est de 0 à 60°C et la pression de 1 à 10 bara. Le courant obtenu en tête de colonne de distillation comprend 5% en poids d'HF, 0,1% de 2-chloro-1,1,1,3,3-pentafluoropropane, 0,2 % de 1,2-dichloro-3,3,3-trifluoropropène et 0,05 % de 2-chloro-1,3,3,3-tetrafluoropropène en poids, le complément étant du 1,1,1,2,2-pentafluoropropane.

### Exemple 2

Un courant comprenant 76,6 % de 1,1,1,2,2-pentafluoropropane, 8% d'HF, 0,7% de 2-chloro-1,1,1,3,3-pentafluoropropane, 0,6 % de 1,2-dichloro-3,3,3-trifluoropropène, 0,5% de 2-chloro-1,3,3,3-tetrafluoropropène, 3% de 1,3,3,3-tetrafluoropropène, 10% de 2-chloro-3,3,3-trifluoropropène, 0,4% de E-1-choro-3,3,3-trifluoropropène, 0,2% de 1,1,1,3,3-pentafluoropropane en poids est distillé dans des conditions telles que la température en tête de colonne de distillation est de 0 à 60°C et la pression de 1 à 10 bara. Le courant obtenu en tête de colonne de distillation comprend 5% en poids d'HF, 0,15 % de 2-chloro-1,1,1,3,3-pentafluoropropane, 0,1 % de 1,2-dichloro-3,3,3-trifluoropropène, 0,03 % de 2-chloro-1,3,3,3-tetrafluoropropène, 3% de 1,3,3,3-tetrafluoropropène, 10% de 2-chloro-3,3,3-trifluoropropène, 0,3% de E-1-choro-3,3,3-trifluoropropène, 0,2% de 1,1,1,3,3-pentafluoropropane en poids en poids, le complément étant du 1,1,1,2,2-pentafluoropropane.

## Revendications

1. Procédé de purification d'un courant incluant 1,1,1,2,2-pentafluoropropane comprenant les étapes de :
i) fourniture d'un courant **A** comprenant 1,1,1,2,2-pentafluoropropane et au moins deux des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
ii) purification, de préférence par distillation, du courant **A** fourni en i) dans des conditions efficaces pour former un premier courant **A1** comprenant 1,1,1,2,2-pentafluoropropane, de préférence récupéré en tête de colonne de distillation, et un second courant **A2** comprenant ledit au moins deux des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène, de préférence récupéré en pied de colonne de distillation.

2. Procédé selon la revendication précédente **caractérisé en ce que** le courant A fourni à l'étape i) et ledit second courant **A2** de l'étape ii) comprennent 1,1,1,2,2-pentafluoropropane, 2-chloro-1,1,1,3,3-pentafluoropropane, 1,2-dichloro-3,3,3-trifluoropropène, 2-chloro-1,3,3,3-tetrafluoropropène.

3. Procédé selon l'une quelconque des revendications précédentes **caractérisé en ce que** de l'acide fluorhydrique est également présent dans ledit courant **A** fourni à l'étape i).

4. Procédé selon l'une quelconque des revendications précédentes **caractérisé en ce que** dans ledit premier courant **A1** obtenu à l'étape ii), le 1,1,1,2,2-pentafluoropropane est obtenu sous la forme d'un mélange azéotrope ou quasi-azéotrope, de préférence un mélange azéotrope ou quasi-azéotrope comprenant 1,1,1,2,2-pentafluoropropane et HF.

5. Procédé selon l'une quelconque des revendications précédentes **caractérisé en ce qu'**il comprend l'addition au courant **A** de l'étape i) d'un flux comprenant au moins 90% de 1,1,1,2,2-pentafluoropropane avant la mise en œuvre de l'étape ii).

6. Procédé selon l'une quelconque des revendications précédentes **caractérisé en ce que** le courant **A** fourni à l'étape i) comprend également (E/Z)-1,3,3,3-tetrafluoropropène, 2-chloro-3,3,3-trifluoropropène, E-1-chloro-3,3,3-trifluoropropène, Z-1-chloro-3,3,3-trifluoropropène ou 1,1,1,3,3-pentafluoropropane.

7. Procédé selon la revendication précédente **caractérisé en ce que** le (E/Z)-1,3,3,3-tetrafluoropropène, 2-chloro-3,3,3-trifluoropropène, E-1-chloro-3,3,3-trifluoropropène ou 1,1,1,3,3-pentafluoropropane est (sont) récupéré(s) dans ledit premier courant **A1** de l'étape (ii).

8. Procédé de production de 2,3,3,3-tetrafluoropropène comprenant les étapes :
a) fluoration en présence d'acide fluorhydrique d'un composé de formule (I) CH₍ₙ₊₂₎(X)ₘ-CHₚ(X)₍ₙ₊₁₎-CX₍₃₊ₚ₋ₘ₎ où X représente indépendamment F ou Cl ; n, m, p sont indépendamment les uns des autres 0 ou 1 avec (n+m) = 0 ou 1, (n+p) = 0 ou 1 et (m-p) = 0 ou 1, au moins un X étant Cl dans des conditions efficaces pour former un courant **C** comprenant 2,3,3,3-tetrafluoropropène, 1,1,1,2,2-pentafluoropropane, et au moins un des composés sélectionné parmi le groupe consistant en 2-chloro-1,1,1,3,3-pentafluoropropane, 1,2-dichloro-3,3,3-trifluoropropène, 2-chloro-1,3,3,3-tetrafluoropropène ;
b) purification, de préférence par distillation, du courant **C** obtenu à l'étape a) pour former un courant **C1** comprenant 2,3,3,3-tetrafluoropropène et un courant **C2** comprenant 1,1,1,2,2-pentafluoropropane, et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène,
c) mise en œuvre du procédé selon l'une quelconque des revendications 1 à 7 à partir du courant **C2** obtenu à l'étape b).

9. Procédé selon la revendication précédente **caractérisé en ce que** le courant **C** obtenu à l'étape a) comprend également HF et ledit courant **C** obtenu à l'étape a) est purifié, de préférence distillé, préalablement à l'étape b) pour former un courant **C'** comprenant 2,3,3,3-tetrafluoropropène, 1,1,1,2,2-pentafluoropropane, et une partie desdits au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène et un courant **C"** comprenant HF et une partie desdits au moins un des composés sélectionné parmi le groupe consistant en 2-chloro-1,1,1,3,3-pentafluoropropane, 1,2-dichloro-3,3,3-trifluoropropène, 2-chloro-1,3,3,3-tetrafluoropropène ; et l'étape b) est mise en œuvre à partir dudit courant **C'.**

10. Procédé selon la revendication précédente **caractérisé en ce que**, dans le courant **C',** la teneur totale en ladite partie desdits au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène est inférieure à 5% en poids sur base du poids total dudit courant **C'.**

11. Procédé de traitement de l'acide fluorhydrique comprenant les étapes de
i') fourniture d'un mélange comprenant de l'acide fluorhydrique et au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène ;
ii') distillation du mélange fourni à l'étape i') dans des conditions efficaces pour former un courant **B1** comprenant ledit au moins un des composés sélectionnés parmi le groupe consistant en 2-chloro-1,1,1,3,3-pentafluoropropane, 1,2-dichloro-3,3,3-trifluoropropène, 2-chloro-1,3,3,3-tetrafluoropropène et un courant **B2** comprenant de l'acide fluorhydrique.

## Patentansprüche

1. Verfahren zur Aufreinigung eines Stoffstroms, der 1,1,1,2,2-Pentafluorpropan beinhaltet, wobei es die folgenden Schritte umfasst:
i) Bereitstellen eines Stoffstroms **A,** der 1,1,1,2,2-Pentafluorpropan und mindestens zwei der Verbindungen umfasst, welche aus der Gruppe ausgewählt sind, die aus 2-Chlor-1,1,1,3,3-pentafluorpropan, 1,2-Dichlor-3,3,3-trifluorpropen, 2-Chlor-1,3,3,3-tetrafluorpropen besteht;
ii) Aufreinigen, vorzugsweise mittels Destillation, des Stoffstroms **A,** wie er in i) bereitgestellt wird, unter Bedingungen, die bewirken, dass sich ein erster Stoffstrom **A1,** welcher 1,1,1,2,2-Pentafluorpropan umfasst, wobei dieser vorzugsweise kopfseitig der Destillationskolonne aufgefangen wird, sowie ein zweiter Stoffstrom **A2** bildet, welcher die mindestens zwei der Verbindungen umfasst, die aus der Gruppe ausgewählt sind, welche aus 2-Chlor-1,1,1,3,3-Pentafluorpropan, 1,2-Dichlor-3,3,3-trifluorpropen, 2-Chlor-1,3,3,3-tetrafluorpropen besteht, wobei dieser vorzugsweise sumpfseitig der Destillationskolonne aufgefangen wird.

2. Verfahren nach dem vorhergehenden Anspruch, **dadurch gekennzeichnet, dass** der Stoffstrom **A,** wie er in Schritt i) bereitgestellt wird, und der zweite Stoffstrom **A2** des Schrittes ii) 1,1,1,2,2-Pentafluorpropan, 2-Chlor-1,1,1,3,3-pentafluorpropan, 1,2-Dichlor-3,3,3-trifluorpropen, 2-Chlor-1,3,3,3-tetrafluorpropen umfassen.

3. Verfahren nach einem beliebigen der vorhergehenden Ansprüche, **dadurch gekennzeichnet, dass** in dem Stoffstrom **A,** wie er in Schritt i) bereitgestellt wird, weiterhin Flusssäure vorliegt.

4. Verfahren nach einem beliebigen der vorhergehenden Ansprüche, **dadurch gekennzeichnet, dass** in dem ersten Stoffstrom **AI,** wie er in Schritt ii) erhalten wird, das 1,1,1,2,2-Pentafluorpropan in Form eines azeotropen oder nahezu azeotropen Gemisches erhalten wird, vorzugsweise eines azeotropen oder nahezu azeotropen Gemisches, das 1,1,1,2,2-Pentafluorpropan und HF umfasst.

5. Verfahren nach einem beliebigen der vorhergehenden Ansprüche, **dadurch gekennzeichnet, dass** im Rahmen desselben dem Stoffstrom **A** des Schrittes i) ein Stofffluss zugesetzt wird, welcher mindestens 90 % an 1,1,1,2,2-Pentafluorpropan umfasst, bevor der Schritt ii) durchgeführt wird.

6. Verfahren nach einem beliebigen der vorhergehenden Ansprüche, **dadurch gekennzeichnet, dass** der Stoffstrom **A,** wie er in Schritt i) bereitgestellt wird, weiterhin (E/Z)-1,3,3,3-Tetrafluorpropen, 2-Chlor-3,3,3-trifluorpropen, E-1-Chlor-3,3,3-trifluorpropen, Z-1-Chlor-3,3,3-trifluorpropen oder 1,1,1,3,3-Pentafluorpropan umfasst.

7. Verfahren nach dem vorhergehenden Anspruch, **dadurch gekennzeichnet, dass** das/die (E/Z)-1,3,3,3-Tetrafluorpropen, 2-Chlor-3,3,3-trifluorpropen, E-1-Chlor-3,3,3-trifluorpropen oder 1,1,1,3,3-Pentafluorpropan aus dem ersten Stoffstrom **A1** des Schrittes (ii) gewonnen wird/werden.

8. Verfahren zur Herstellung von 2,3,3,3-Tetrafluorpropen, das die folgenden Schritte umfasst:
a) Fluorieren, in Gegenwart von Flusssäure, einer Verbindung der Formel (I) CH₍ₙ₊₂₎(X)ₘ-CHₚ(X)₍ₙ₊₁₎-CX₍₃₊ₚ₋ₘ₎, wobei X auf unabhängige Weise für F oder C1 steht; n, m, p unabhängig voneinander gleich 0 oder 1 sind, mit (n+m) = 0 oder 1, (n+p) = 0 oder 1 und (m-p) = 0 oder 1, wobei mindestens ein X für Cl steht, unter Bedingungen, die bewirken, dass sich ein Stoffstrom **C** bildet, der 2,3,3,3-Tetrafluorpropen, 1,1,1,2,2-Pentafluorpropan und mindestens eine der Verbindungen umfasst, welche aus der Gruppe ausgewählt sind, die aus 2-Chlor-1,1,1,3,3-pentafluorpropan, 1,2-Dichlor-3,3,3-trifluorpropen, 2-Chlor-1,3,3,3-tetrafluorpropen besteht;
b) Aufreinigen, vorzugsweise mittels Destillation, des Stoffstroms **C,** der im Schritt a) erhalten wurde, um einen Stoffstrom **C1,** welcher 2,3,3,3-Tetrafluorpropen umfasst, sowie einen Stoffstrom **C2** zu erhalten, welcher 1,1,1,2,2-Pentafluorpropan und mindestens eine der Verbindungen umfasst, die aus der Gruppe ausgewählt sind, welche aus 2-Chlor-1,1,1,3,3-pentafluorpropan, 1,2-Dichlor-3,3,3-trifluorpropen, 2-Chlor-1,3,3,3-tetrafluorpropen besteht,
c) Durchführen des Verfahrens nach einem beliebigen der Ansprüche 1 bis 7 ausgehend von dem Stoffstrom **C2,** welcher in Schritt b) erhalten wurde.

9. Verfahren nach dem vorhergehenden Anspruch, **dadurch gekennzeichnet, dass** der Stoffstrom **C,** welcher in Schritt a) erhalten wurde, weiterhin HF umfasst und der Stoffstrom **C,** welcher in Schritt a) erhalten wurde, im Vorfeld des Schrittes b) aufgereinigt wird, nämlich vorzugsweise destilliert wird, um einen Stoffstrom **C',** welcher 2,3,3,3-Tetrafluorpropen, 1,1,1,2,2-Pentafluorpropan sowie eine Teilmenge der Verbindungen umfasst, von denen mindestens eine vorliegt und die aus der Gruppe ausgewählt sind, welche aus 2-Chlor-1,1,1,3,3-pentafluorpropan, 1,2-Dichlor-3,3,3-trifluorpropen, 2-Chlor-1,3,3,3-tetrafluorpropen besteht, sowie einen Stoffstrom **C"** zu bilden, welcher HF und eine Teilmenge der Verbindungen umfasst, von denen mindestens eine vorliegt und die aus der Gruppe ausgewählt sind, welche aus 2-Chlor-1,1,1,3,3-pentafluorpropan, 1,2-Dichlor-3,3,3-trifluorpropen, 2-Chlor-1,3,3,3-tetrafluorpropen besteht; und der Schritt b) ausgehend von dem Stoffstrom **C'** durchgeführt wird.

10. Verfahren nach dem vorhergehenden Anspruch, **dadurch gekennzeichnet, dass** im Stoffstrom **C'** der Gesamtgehalt an der Teilmenge der Verbindungen, von denen mindestens eine vorliegt und die aus der Gruppe ausgewählt sind, welche aus 2-Chlor-1,1,1,3,3-pentafluorpropan, 1,2-Dichlor-3,3,3-trifluorpropen, 2-Chlor-1,3,3,3-tetrafluorpropen besteht, geringer als 5 Gewichts-% ist, bezogen auf das Gesamtgewicht des Stoffstroms **C'.**

11. Verfahren zur Behandlung von Flusssäure, das die folgenden Schritt umfasst
i') Bereitstellen einer Mischung, die Flusssäure und mindestens eine der Verbindungen umfasst, welche aus der Gruppe ausgewählt sind, die aus 2-Chlor-1,1,1,3,3-pentafluorpropan, 1,2-Dichlor-3,3,3-trifluorpropen, 2-Chlor-1,3,3,3-tetrafluorpropen besteht;
ii') Destillieren der Mischung, wie sie in Schritt i') bereitgestellt wird, unter Bedingungen, die bewirken, dass sich ein Stoffstrom **B1,** welcher die mindestens eine der Verbindungen enthält, die aus der Gruppe ausgewählt sind, welche aus 2-Chlor-1,1,1,3,3-pentafluorpropan, 1,2-Dichlor-3,3,3-trifluorpropen, 2-Chlor-1,3,3,3-tetrafluorpropen besteht, sowie ein Stoffstrom **B2** bildet, welcher Flusssäure umfasst.

## Claims

1. Process for purifying a stream including 1,1,1,2,2-pentafluoropropane, comprising the steps of:
i) providing a stream **A** comprising 1,1,1,2,2-pentafluoropropane and at least two of the compounds selected from the group consisting of 2-chloro-1,1,1,3,3-pentafluoropropane, 1,2-dichloro-3,3,3-trifluoropropene, 2-chloro-1,3,3,3-tetrafluoropropene;
ii) purification, preferably by distillation, of the stream **A** provided in i) under conditions effective for forming a first stream **A1** comprising 1,1,1,2,2-pentafluoropropane, preferably recovered at the top of the distillation column, and a second stream **A2** comprising said at least two of the compounds selected from the group consisting of 2-chloro-1,1,1,3,3-pentafluoropropane, 1,2-dichloro-3,3,3-trifluoropropene, 2-chloro-1,3,3,3-tetrafluoropropene, preferably recovered at the bottom of the distillation column.

2. Process according to the preceding claim, **characterized in that** the stream **A** provided in step i) and said second stream **A2** of step ii) comprise 1,1,1,2,2-pentafluoropropane, 2-chloro-1,1,1,3,3-pentafluoropropane, 1,2-dichloro-3,3,3-trifluoropropene, 2-chloro-1,3,3,3-tetrafluoropropene.

3. Process according to any one of the preceding claims, **characterized in that** the hydrofluoric acid is also present in said stream **A** provided in step i).

4. Process according to any one of the preceding claims, **characterized in that** in said first stream **A1** obtained in step ii), the 1,1,1,2,2-pentafluoropropane is obtained in the form of an azeotropic or quasi-azeotropic mixture, preferably an azeotropic or quasi-azeotropic mixture comprising 1,1,1,2,2-pentafluoropropane and HF.

5. Process according to any one of the preceding claims, **characterized in that** it comprises the addition, to the stream **A** of step i), of a flow comprising at least 90% of 1,1,1,2,2-pentafluoropropane before carrying out step ii).

6. Process according to any one of the preceding claims, **characterized in that** the stream **A** provided in step i) also comprises (E/Z)-1,3,3,3-tetrafluoropropene, 2-chloro-3,3,3-trifluoropropene, E-1-chloro-3,3,3-trifluoropropene, Z-1-chloro-3,3,3-trifluoropropene or 1,1,1,3,3-pentafluoropropane.

7. Process according to the preceding claim, **characterized in that** the (E/Z)-1,3,3,3-tetrafluoropropene, 2-chloro-3,3,3-trifluoropropene, E-1-chloro-3,3,3-trifluoropropene or 1,1,1,3,3-pentafluoropropane is (are) recovered in said first stream **A1** of step (ii).

8. Process for producing 2,3,3,3-tetrafluoropropene, comprising the steps of:
a) fluorination, in the presence of hydrofluoric acid, of a compound of formula (I) CH₍ₙ₊₂₎(X)ₘ-CHₚ(X)₍ₙ₊₁₎-CX₍₃₊ₚ₋ₘ₎ in which X represents independently F or Cl; n, m, p are independently of one another 0 or 1 with (n+m) = 0 or 1, (n+p) = 0 or 1 and (m-p) = 0 or 1, at least one X being Cl, under conditions effective for forming a stream **C** comprising 2,3,3,3-tetrafluoropropene, 1,1,1,2,2-pentafluoropropane, and at least one of the compounds selected from the group consisting of 2-chloro-1,1,1,3,3-pentafluoropropane, 1,2-dichloro-3,3,3-trifluoropropene, 2-chloro-1,3,3,3-tetrafluoropropene;
b) purification, preferably by distillation, of the stream **C** obtained in step a) in order to form a stream **C1** comprising 2,3,3,3-tetrafluoropropene and a stream **C2** comprising 1,1,1,2,2-pentafluoropropane, and at least one of the compounds selected from the group consisting of 2-chloro-1,1,1,3,3-pentafluoropropane, 1,2-dichloro-3,3,3-trifluoropropene, 2-chloro-1,3,3,3-tetrafluoropropene,
c) carrying out the process according to any one of Claims 1 to 7 using the stream **C2** obtained in step b).

9. Process according to the preceding claim, **characterized in that** the stream **C** obtained in step a) also comprises HF and said stream **C** obtained in step a) is purified, preferably distilled, prior to step b) in order to form a stream **C'** comprising 2,3,3,3-tetrafluoropropene, 1,1,1,2,2-pentafluoropropane, and a portion of said at least one of the compounds selected from the group consisting of 2-chloro-1,1,1,3,3-pentafluoropropane, 1,2-dichloro-3,3,3-trifluoropropene, 2-chloro-1,3,3,3-tetrafluoropropene and a stream **C"** comprising HF and a portion of said at least one of the compounds selected from the group consisting of 2-chloro-1,1,1,3,3-pentafluoropropane, 1.2-dichloro-3.3.3-trifluoropropene, 2-chloro-1,3,3,3-tetrafluoropropene; and step b) is carried out using said stream **C'.**

10. Process according to the preceding claim, **characterized in that**, in the stream **C'**, the total content of said portion of said at least one of the compounds selected from the group consisting of 2-chloro-1,1,1,3,3-pentafluoropropane, 1,2-dichloro-3,3,3-trifluoropropene, 2-chloro-1,3,3,3-tetrafluoropropene is less than 5% by weight based on the total weight of said stream **C'.**

11. Process for treating hydrofluoric acid, comprising the steps of
i') providing a mixture comprising hydrofluoric acid and at least one of the compounds selected from the group consisting of 2-chloro-1,1,1,3,3-pentafluoropropane, 1.2-dichloro-3.3.3-trifluoropropene, 2-chloro-1,3,3,3-tetrafluoropropene;
ii') distillation of the mixture provided in step i') under conditions effective for forming a stream **B1** comprising said at least one of the compounds selected from the group consisting of 2-chloro-1,1,1,3,3-pentafluoropropane, 1,2-dichloro-3,3,3-trifluoropropene, 2-chloro-1,3,3,3-tetrafluoropropene and a stream **B2** comprising hydrofluoric acid.
